# FASCICULE DE BREVET EUROPEEN

(11) **EP 3 463 100 B1**
(45) Date de publication et mention de la délivrance du brevet: **28.09.2022**
(21) Numéro de dépôt: 17727266.3
(22) Date de dépôt: 07.06.2017
(51) Int. Cl.: A61B 10/00

(54) **DISPOSITIF DE PRISE D'ÉCHANTILLON INTESTINAL**
VORRICHTUNG ZUR ENTNAHME VON DARMFLÜSSIGKEITSPROBEN
APPARATUS FOR TAKING INTESTINAL SAMPLES

(30) Priorité: 07.06.2016 FR 1655187
(43) Date de publication de la demande: 10.04.2019
(73) Titulaire: Université Grenoble Alpes, 38401 Saint-Martin-d'Hères (FR); Centre Hospitalier Universitaire Grenoble, 38043 La Tronche (FR)
(72) Inventeur: CINQUIN, Philippe, 38330 Saint Nazaire les Eymes (FR); FAVIER, Denis, 38700 La Tronche (FR); ALONSO, Thierry, 38330 St Ismier (FR); BAKRI, Aziz, 38100 Grenoble (FR); KHALEF, Nawel, 38240 Meylan (FR); THELU, Jacques, 38920 Crolles (FR); BESSON, Sylvain, 38190 Villard-Bonnot (FR); MARTIN, Donald, 38610 Gieres (FR)
(74) Mandataire: Hautier IP
(86) Numéro de dépôt international: PCT/EP2017/063791
(87) Numéro de publication internationale: WO 2017/211872

(56) Documents cités:
- WO-A1-79/00811
- WO-A1-2005/046485
- DE-A1- 19 801 573
- US-A1- 2007 173 738
- US-A1- 2016 038 086

## Description

La présente invention est relative à un dispositif permettant la prise d'échantillon dans l'intestin, notamment à des fins de recherche et de diagnostic.

A l'heure où l'on considère le système digestif et son microbiote comme un sujet d'intérêt majeur en santé humaine, notamment en termes de diagnostic de pathologies, où l'on développe sans cesse de nouveaux probiotiques sans réellement connaître ou être en mesure de connaître leurs effets tout au long de l'intestin, il existe un besoin en un moyen permettant de faire des prises d'échantillon représentatives de parties déterminées du tractus.

US 5,971,942 décrit des capsules de prise d'échantillon formées d'une enveloppe délimitant un volume intérieur dans lequel règne une pression basse, et munie d'une ouverture obturée par un joint apte à se dissoudre dans le milieu intestinal, le liquide intestinal étant alors aspiré à l'intérieur de la capsule par la différence de pression, et la fermeture de la capsule étant obtenu par l'équilibre des pressions hors et dans la capsule. Des capsules à plusieurs compartiments ou l'emploi de plusieurs capsules ayant des joints à vitesse de dissolution différentes peuvent être utilisées pour prélever des échantillons à divers endroits. Ces capsules ne comprennent pas de mécanisme mobile d'ouverture et/ou de fermeture de l'ouverture commandé par la dissolution d'un matériau dissolvable dans l'intestin. Ces capsules sont limitées en volume intérieur et nécessitent une basse pression intérieure à la fabrication.

WO 79/00811 A1 divulgue un dispositif d'échantillonnage pour prélever automatiquement des échantillons de liquides gastriques et intestinaux et pour déterminer la présence d'acide chlorhydrique et d'acide lactique dans le tube digestif des humains ou des animaux.

US 2016/0038086 A1 divulgue un dispositif pour échantillonner des substances dans le tube digestif d'animaux, le dispositif comprenant au moins une paroi partiellement étendable.

La présente invention a pour objectif de fournir un dispositif permettant le prélèvement d'échantillons à différents niveaux dans le tractus intestinal, et ce de préférence de manière prédéterminée.

Un autre objectif de l'invention est de proposer un tel dispositif qui permette de recueillir des volumes variables, tout en restant facilement avalable par le patient.

Un autre objectif de l'invention est de proposer un tel dispositif qui soit d'un coût de revient acceptable et facilement utilisable et récupérable.

L'invention a donc pour objet une capsule destinée au prélèvement d'échantillon dans l'intestin (intestin grêle ou gros intestin), tel que défini dans la revendication indépendante 1.

La capsule comprend un corps délimitant au moins un compartiment de réception d'échantillon et au moins une ouverture d'admission d'échantillon (liquide ou matière intestinale) associée à un mécanisme d'ouverture et/ou de fermeture commandé par la dissolution d'un matériau dégradable/dissolvable dans l'intestin. L'entrée d'un échantillon par l'ouverture peut notamment se faire de manière passive par ouverture mécanique ou par aspiration par déploiement mécanique du compartiment.

La capsule comprend un matériau dissolvable dans l'intestin (ce qui signifie au contact du contenu de l'intestin), qui entoure partiellement ou complètement le corps de la capsule et dont la dissolution libère l'ouverture ou fait qu'un mécanisme provoque cette ouverture et/ou une aspiration. La capsule peut comprendre ou loger un dispositif mobile de fermeture de l'ouverture qui, à l'état initial, est immobilisé par un frein en matériau dissolvable dans l'intestin, et, après dissolution de ce frein, s'actionne pour fermer l'ouverture.

De manière remarquable, des dispositifs de l'invention présentent une variation de leur volume extérieur et intérieur suite à la dissolution ou dégradation d'un matériau les contraignant initialement dans une forme de volume réduit.

De manière tout aussi remarquable, des dispositifs de l'invention comprennent plusieurs compartiments qui sont mis en relation avec l'ouverture de manière successive.

Suivant une caractéristique de l'invention, la capsule peut être entourée d'un matériau gastro-résistant, permettant le passage sans encombre de l'estomac. Une fois ce matériau dissout à l'intérieur de l'intestin, la capsule peut fonctionner. Le liquide intestinal peut dissoudre un matériau dégradable obturant une ouverture donnant sur l'intérieur du compartiment, et/ou peut dissoudre un matériau dégradable contraignant le corps sous une forme repliée, la dégradation libérant le corps qui peut se déployer et s'ouvrir au liquide intestinal, et/ou dissoudre un frein ou analogue afin de libérer le dispositif mobile d'ouverture et/ou fermeture. L'homme du métier comprendra aisément que l'on peut jouer sur le choix du matériau et sa vitesse de dissolution dans l'intestin pour déterminer le moment où la capsule est prête à entrer en fonction. On peut prévoir plus d'une couche de matériau, une couche de matériau extérieure pour passer la barrière de l'estomac, et au moins une couche intérieure pour prolonger le temps de latence intra-intestinal avant que la capsule n'entre en fonction.

L'invention concerne donc une capsule destinée au prélèvement d'échantillon dans l'intestin, caractérisée en ce qu'elle comprend un corps délimitant au moins un compartiment de réception d'échantillon et comportant au moins une ouverture d'admission d'échantillon, et un mécanisme mobile d'ouverture et/ou de fermeture de l'ouverture commandé par la dissolution d'un matériau dissolvable dans l'intestin. Le corps de la capsule est entouré totalement ou partiellement d'un matériau dissolvable (y compris plusieurs matériaux, comme il sera expliqué plus loin, notamment un premier gastro-résistant et un matériau dissolvable dans l'intestin après un temps de séjour déterminé) dans l'intestin dont la dissolution déclenche ou libère le mécanisme mobile d'ouverture et de fermeture de l'ouverture.

Dans un mode de réalisation, le corps de la capsule peut être contraint dans une ou plusieurs directions sous une forme repliée à l'intérieur d'un matériau dégradable dans l'intestin. Une fois ce matériau dissout à l'intérieur de l'intestin, la capsule (le corps de la capsule) est libérée et se déploie pour prendre sa forme active, offrant une ouverture et un volume de compartiment accru par rapport à la forme non déployée. Ceci présente aussi l'avantage de disposer d'une capsule repliée facilement avalable par l'individu ou de réduire tellement le volume qu'il est possible d'en avaler plusieurs à la fois, notamment réunies dans un même contenant (gélule ou capsule, qui peut du même coup former le matériau gastro-résistant). Suivant une modalité, on peut disposer d'une série de capsules repliées, de préférence réunies dans une même gélule ou capsule plus grosse, auxquelles un matériau dégradable dans l'intestin confère des vitesses de dissolution dans l'intestin différentes et prédéterminées, permettant de recueillir des échantillons à différents instants du transit dans l'intestin. De préférence alors, les capsules ont des marqueurs de reconnaissance, par exemple une couleur ou autre signe permettant de les classer après recueil.

Dans un mode de réalisation, la capsule est de forme cylindrique ou sensiblement cylindrique. Avantageusement, conformément au paragraphe précédent, son corps peut se déployer axialement et/ou de manière rayonnée autour de l'axe du cylindre.

Dans une première forme de réalisation, la capsule est du type dont le corps est initialement contraint dans un matériau dissolvable dans l'intestin, et possède une forme déployée cylindrique entre deux disques rigides réunis par un ressort ou un stent les écartant. La capsule peut comporter une membrane étanche et extensible reliée aux disques pour former la surface du cylindre. Initialement la membrane s'étend sur une partie de la circonférence du cylindre et l'un de ses bords libres, de préférence renforcé par un jonc extensible ou souple (e.g. corde) solidaire d'un ressort conçu pour déployer la membrane extensible jusqu'à fermer entièrement le cylindre, ledit ressort étant initialement contraint par un frein en matériau dissolvable dans l'intestin. De préférence, les deux disques sont munis sur leur tranche d'une rainure coopérant chacune avec un ressort raccordé au jonc, pour entraîner la membrane étanche.

Dans une deuxième forme de réalisation, le corps de la capsule est contraint dans deux directions orthogonales sous une forme repliée à l'intérieur du matériau dissolvable dans l'intestin. De forme cylindrique, elle se déploie axialement et de manière rayonnée autour de l'axe du cylindre.

La capsule peut comprendre, contrainte dans le matériau dissolvable dans l'intestin, au moins une structure maillée tubulaire extensible dans les deux directions orthogonales, notamment du type stent maillé, éventuellement en matériau à mémoire de forme ou superélastique, e.g. en alliage Ni-Ti. Avantageusement, cette structure maillée est cylindrique. Ses deux bases peuvent être solidaires de deux disques en matériau extensible (par exemple élastomère) pour accompagner l'extension rayonnée de la structure maillée. Lorsque la capsule se déploie, les deux disques s'écartent l'un de l'autre sous l'effet de l'extension axiale de la structure maillée. Chacun des disques peut être associé à une paroi cylindrique en matériau extensible s'étendant depuis le bord du deuxième disque (ou proche du bord du disque). Lorsque le déploiement complet de la capsule est atteint et l'échantillon entré dans le compartiment, les deux parois cylindriques viennent au contact l'une de l'autre, de préférence se chevauchent au moins en partie, et ferment la capsule.

Dans une première modalité, l'une des bases de la structure maillée tubulaire peut être en appui sur un premier disque en matériau extensible solidaire. Ce premier disque peut être solidaire d'une paroi cylindrique en matériau extensible s'étendant depuis le bord du disque (ou proche du bord du disque), à l'intérieur de la structure maillée tubulaire sur une partie de la surface de celle-ci. L'autre base de la structure maillée tubulaire peut être en appui sur un deuxième disque en matériau extensible. Ce deuxième disque peut être solidaire d'une paroi cylindrique en matériau extensible s'étendant depuis le bord du deuxième disque (ou proche du bord du disque), à l'intérieur de la structure maillée tubulaire. Cette paroi n'est pas déployée au repos. Elle peut être associée à un ressort de déploiement contraint en position non déployée par un matériau dissolvable au contact du contenu de l'intestin et conçu pour, après dissolution de ce matériau formant frein, déployer la paroi associée au deuxième disque jusqu'à chevaucher au moins en partie la paroi associée au premier disque.

Dans une deuxième modalité, l'une des bases de la structure maillée tubulaire peut être en appui sur un premier disque en matériau extensible. Ce premier disque peut être solidaire d'une paroi cylindrique en matériau extensible s'étendant depuis le bord du disque (ou proche du bord du disque), à l'extérieur de la structure maillée tubulaire sur une partie de la surface de celle-ci. L'autre base de la structure maillée tubulaire peut être en appui sur un deuxième disque en matériau extensible. Ce deuxième disque peut être solidaire d'une paroi cylindrique en matériau extensible s'étendant depuis le bord du deuxième disque (ou proche du bord du disque), à l'extérieur de la structure maillée tubulaire. On peut prévoir un dispositif permettant de contraindre la structure maillée axialement au-delà de sa position déployée de repos, et dimensionner les parois cylindriques pour que, dans cette position, se découpe une ouverture circonférentielle permettant l'entrée de liquide intestinal dans le compartiment. Ce dispositif peut être relié aux disques par un matériau dissolvable dans l'intestin, si bien qu'une fois la dissolution faite, la structure maillée prend sa forme déployée de repos fermée par les parois. Il peut s'agir d'un ensemble de deux ressorts en appui décalé sur un disque auquel ces ressorts sont collés à l'aide d'une colle dissolvable avec le liquide intestinal, si bien qu'après dissolution l'appui décalé fait que les ressorts s'échappent l'un de l'autre, et la structure maillée prend sa forme déployée de repos, et les parois cylindriques viennent en contact (de préférence en se chevauchant au moins en partie) pour fermer le compartiment.

Dans un autre mode de réalisation, le corps de la capsule loge une structure en forme d'aube comportant des parois délimitant des compartiments rayonnés à partir d'un axe central, la structure étant solidaire d'un ressort, par exemple un fil ressort de torsion, ou d'un dispositif mécanique la sollicitant en rotation. Au moins un frein dissolvable au contact du contenu de l'intestin peut être associé à chaque compartiment pour ne déclencher la rotation et le passage du compartiment devant l'ouverture qu'après dissolution de ce frein. Le ressort ou le dispositif mécanique peut exercer une contrainte constante, la rotation étant empêchée par la présence d'une butée contre laquelle vient s'appuyer un frein dissolvable. Lorsque le frein dissolvable d'un compartiment en appui sur la butée est dissout ou dégradé, la butée est libérée et la structure en forme d'aube tourne d'un pas jusqu'à la rencontre entre la butée et le frein suivant, si bien que ledit compartiment est alors placé devant l'ouverture et l'échantillon peut entrer. A la dissolution du frein du compartiment suivant, la structure tourne d'un pas et c'est le compartiment suivant qui se retrouve face à l'ouverture, ainsi de suite. L'ensemble des freins peut être au contact du liquide intestinal, les freins ayant des vitesses de dégradation prédéterminées et se réduisant depuis le frein associé à l'ouverture du premier compartiment et celui associé au dernier compartiment. Ceci peut être obtenu par le choix du matériau ou par l'épaisseur du frein.

Dans un mode de réalisation, le corps de la capsule est formé d'une cage comprenant une structure de base entourée d'une membrane étanche souple et élastique, par exemple en latex ou en matériau silicone, la cage ayant naturellement (au repos, déployée) une forme ovoïde entre deux extrémités. Il peut notamment s'agir d'une cage en matériau à mémoire de forme ou superélastique, e.g. en alliage NiTi. La cage est initialement contrainte par un matériau, notamment une ou plusieurs bandes de matériau, dégradable dans l'intestin, disposé(es) autour de la membrane étanche, et de préférence la forme non déployée est cylindrique. La structure présente en outre une ouverture à l'une de ses extrémités ou une ouverture à chacune de ses extrémités. La dissolution ou la dégradation de la ou les bandes de matériau dégradable permet à la structure de se déployée et d'aspirer le liquide intestinal à son intérieur.

Suivant une première modalité, au niveau d'une extrémité ou des deux extrémités, on dispose une valve anti-retour, permettant l'aspiration de liquide dans la cage lorsque celle-ci se déploie, la valve empêchant la sortie du liquide une fois la cage remplie. Tout type de valve anti-retour peut être employé, comportant une pièce mobile permettant l'ouverture de l'entrée d'admission de liquide lorsque la cage se déploie, et obturant cette entrée lorsque la cage est remplie. A titre d'exemple simple, la membrane souple est retournée sur elle-même comme un doigt de gant, vers l'intérieur de la cage, formant une valve anti-retour. Le liquide intestinal est aspiré à l'intérieur de la capsule au moment du gonflement de la cage, il passe librement l'ouverture en écartant la membrane. Une fois la capsule remplie, le liquide ne peux ressortir puisque l'élasticité et la forme de la membrane constitue cette fois une barrière au liquide.

Suivant une deuxième modalité, les deux ouvertures sont associées à un mécanisme mobile d'ouverture et fermeture, avec un tampon d'étanchéité associé audit mécanisme mobile pour assurer la fermeture de ces ouvertures seulement lorsque la structure s'est déployée. Ces tampons peuvent avantageusement être montés sur une tige s'étendant axialement au travers de la structure et des ouvertures et coopérant avec des paliers placés dans ces ouvertures. Notamment, le mécanisme comprend une tige s'étendant axialement au travers de la structure, des paliers placés dans ces ouvertures, chaque palier ayant un orifice central de diamètre supérieur au diamètre de la tige, les tampons d'étanchéité étant montés sur la tige à l'intérieur de la structure maillée à un emplacement les amenant en appui sur les paliers lorsque la structure a pris sa forme ovoïde. L'ouverture d'admission est formée par l'espace laissé par la tige dans l'orifice central des paliers, et le déploiement de la structure mailée aspire l'échantillon par cet espace, jusqu'à ce que les tampons viennent en appui sur les paliers.

Le corps peut être formé d'une cage comprenant une structure de base formée de barreaux entourés d'une membrane étanche souple et élastique, la cage ayant naturellement (au repos) une forme ovoïde. La cage est contrainte notamment sous forme cylindrique par tout moyen approprié, par exemple une ou plusieurs bandes de matériau dissolvable au contact du liquide intestinal

En variante, la cage est en structure maillée de type stent à mémoire de forme, la cage ayant naturellement (déployée au repos) une forme ovoïde.

Ce mode de réalisation est particulièrement, mas pas exclusivement, adapté au recueil d'échantillons de volume relativement grand, notamment compris entre environ 0,5 mL et environ 2 mL.

Par matériau dégradable ou dissolvable (on emploie dans le texte indifféremment un mot ou l'autre, sauf indication contraire), on entend différents types de mécanismes chimiques, physico-chimiques et/ou enzymatiques.

Le matériau peut être intégralement soluble, ou un ou plusieurs composant(s) du matériau peut/peuvent être soluble(s). Ainsi, la solubilité ou la dégradation/digestion du matériau peut être obtenue par dissolution d'un composant principal tel qu'un polymère ou d'un composant tel qu'un liant, conduisant à une dissolution totale ou partielle du matériau, suffisant pour libérer la capsule de ce matériau. On peut aussi avoir, à la place ou en plus, un mécanisme enzymatique avec digestion d'un composant principal tel qu'un polymère (ou un mélange de polymères) ou d'un composant tel qu'un liant, conduisant à une digestion totale ou partielle du matériau, suffisant pour libérer la capsule de ce matériau. Ces matériaux sont aptes à subir cette dissolution ou dégradation dans les conditions physiologiques de l'intestin. Comme matériaux dégradables/dissolvables dans l'intestin on pourra mentionner des polymères synthétiques tels que la PVA (alcool polyvinylique), les Poly(acrylic acid) (Carbomer), les oxydes de Polyethylène , les Polymethacrylates, poly lactic / glycolic acid (PLGA)... Des polymères naturels tels que les polysaccharides : Agarose, Chitosan, Alginates, ou les protéines tels que la gélatine ou encore semi synthétique tels que les dérivés cellulosiques : Hydroxypropyl méthyl cellulose (HPMC), Hydroxyde éthyle cellulose (HEC), et Carboxyle méthyle cellulose (CMC), la gélatine, les triglycérides (cire)... Des vitesses de dégradation différentes peuvent être obtenues comme cela est connu de l'homme du métier, par exemple par le choix du matériau, par exemple nature du matériau, du polymère, présence ou pas d'additifs jouant sur la solubilité du matériau, du polymère ou d'un composant, par exemple un liant, composition pondérale lorsqu'il y a plusieurs composants, nature des enzymes de dégradation dans le cas où la dégradation se fait de manière enzymatique, et/ou l'épaisseur du matériau.

Comme matériaux gastro-résistants, on peut mentionner l'Acétophthalate de cellulose (CAP), la Carboxymethyl cellulose (CMC), l'Hypromellose Acetate Succinate (HPMCAS), les Polymetacrylates, les Polyvinyl Acetate Phthalate (PVAP), les gommes laque (shellac)...

Par alliage ou métal à mémoire de forme, on entend un matériau qui a été conditionné par les méthodes connues de l'homme du métier, pour prendre une certaine forme à 37°C en l'absence d'autre contrainte mécanique exercée sur lui.

Plusieurs petites capsules peuvent être réunies ensemble dans une gélule ou grande capsule, comme il a été dit. Il est aussi possible de disposer d'un chapelet de plusieurs petites capsules, reliées par cordon ou collées ensemble par un matériau ne se dissolvant pas dans l'estomac et dans l'intestin.

Des marqueurs colorés ont été mentionnés pour permettre la reconnaissance des capsules récupérées. On peut utiliser aussi une puce en silicium, une puce RFID ou analogue.

Les capsules peuvent aussi comporter une partie métallique permettant de les localiser à l'intérieur du corps et/ou faciliter leur récupération dans les fèces à l'aide d'un aimant ou autre dispositif magnétique.

L'invention va être maintenant décrite plus en détail à l'aide de modes de réalisation pris à titre d'exemples non limitatifs se référant au dessin annexé dans lequel :
- La figure 1 représente une vue schématique de trois-quarts d'une capsule cylindrique contrainte dans un enrobage dissolvable dans l'intestin selon un premier mode de réalisation.
- Les figures 2-4 représentent des vues schématiques de la capsule de la figure 1, déployée après dissolution de l'enrobage, dans des configurations successives ouverte (figure 2), en cours de fermeture (figure 3) et fermée (figure 4). Ces vues sont en coupe selon un plan contenant l'axe (horizontal) de la capsule.
- La figure 5 représente une vue schématique d'une capsule maillée contrainte dans un enrobage dissolvable dans l'intestin selon un deuxième mode de réalisation.
- Les figures 6 et 7 représentent des vues schématiques de la capsule de la figure 5, déployée après dissolution de l'enrobage, dans des configurations successives ouverte (figure 6) et fermée (figure 7). Ces vues sont en coupe selon un plan contenant l'axe (horizontal) de la capsule.
- La figure 8 représente une vue schématique d'une capsule maillée contrainte dans un enrobage dissolvable dans l'intestin selon un troisième mode de réalisation.
- Les figures 9 et 10 représentent des vues schématiques de la capsule de la figure 8, déployée après dissolution de l'enrobage, dans des configurations successives ouverte (figure 9) et fermée (figure 10).
- La figure 11 représente une vue schématique éclatée d'une capsule selon un quatrième mode de réalisation.
- La figure 12 est une représentation schématique en élévation de la partie de la capsule de la figure 11, délimitant six compartiments.
- La figure 13 représente une vue schématique en élévation d'une capsule selon un cinquième mode de réalisation.
- La figure 14 représente une vue schématique en coupe de la capsule de la figure 13, en coupe selon un plan contenant l'axe (horizontal) de la capsule, avant et après déploiement de la capsule et aspiration d'échantillon, suivant une première modalité.
- La figure 15 représente une vue schématique en coupe de la capsule de la figure 13, en coupe selon un plan contenant l'axe (horizontal) de la capsule, suivant une deuxième modalité.
- La figure 16 représente une vue schématique en élévation d'une capsule selon un sixième mode de réalisation.

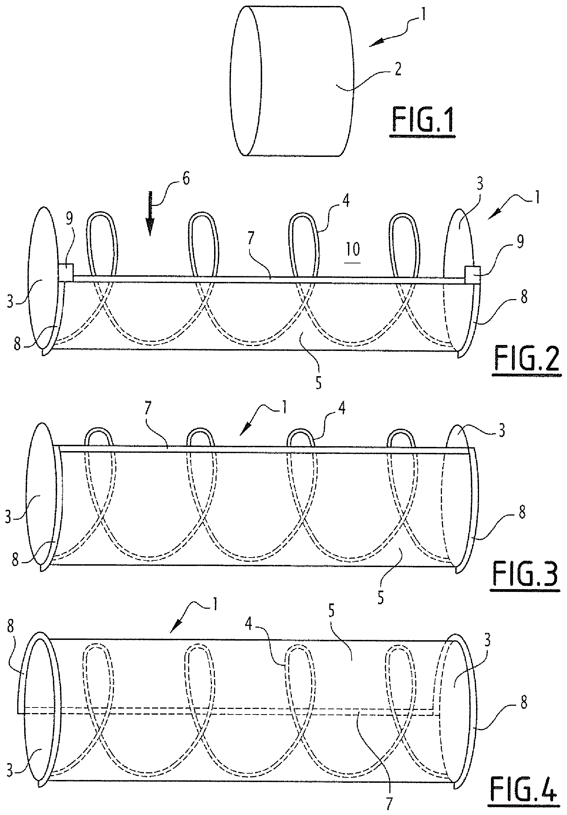

La figure 1 représente une vue schématique de trois-quarts d'une capsule 1 contrainte dans un enrobage 2 dissolvable dans l'intestin selon un premier mode de réalisation. L'ensemble forme un cylindre dont les dimensions vont pouvoir varier, selon différentes formes de réalisation.

Dans une première forme de réalisation, la capsule du type de celle représentée à la figure 1 a les dimensions suivantes : de 1 à 6 mm, par exemple 4 mm de diamètre, et de 1 à 6 mm, par exemple 4 mm de longueur. Grâce à ces dimensions réduites, plusieurs, par exemple 5, 10, 15 ou 20, de ces capsules peuvent être contenues dans une grande capsule de plus grandes dimensions, par exemple de longueur 25 mm et 12 mm de diamètre, apte à être prise par le patient par voie orale.

Dans une deuxième forme de réalisation, la capsule du type de celle représentée à la figure 1 a les dimensions suivantes : 6 à 20 mm, par exemple 12 mm de diamètre, et de 10 à 30 mm, par exemple 25 mm de longueur. Grâce à ces dimensions chaque capsule ainsi constituée est apte à être prise par le patient par voie orale.

Comme on peut le voir aux figures 2-4, après dissolution de l'enrobage 2, la capsule 1 se déploie. La capsule 1 a une forme déployée cylindrique entre deux disques 3 rigides réunis par un premier ressort 4 les écartant, chacun des disques ayant une gorge circulaire (non représentée) sur sa tranche. La capsule comporte une membrane 5 étanche et extensible reliée aux disques 3 pour former la surface du cylindre. Initialement (lorsque la capsule 1 s'est déployée après dissolution de l'enrobage 2) la membrane 5 s'étend sur une partie de la circonférence du cylindre, la partie libre formant l'ouverture d'admission de l'échantillon figurée par la flèche 6. L'un des bords libres de la membrane 5 est solidaire d'une corde 7 extensible et solidaire de deux ressorts cylindriques (hélicoïdaux) 8 disposés chacun dans une gorge d'un disque 3. Ces ressorts 8, lorsqu'ils se détendent après avoir été libérés, sont conçus pour entraîner la corde 7 et déployer la membrane 5 extensible progressivement et jusqu'à fermer entièrement le cylindre, les ressorts 8 entraînant la corde 7 dans un mouvement de rotation guidé dans les gorges des disques 3. Au repos, ces ressorts 8 sont comprimés et bloqués par deux freins 9 en matériau dissolvable au contact du contenu intestinal, chacun des freins étant disposé aux extrémités de la corde 7 au contact des disques 3. On voit à la figure 3 que les freins 9 ont disparu, dissous au contact du liquide intestinal, et que la corde 7, sous l'action des ressorts 8, a commencé à fermer l'espace intérieur ou compartiment 10 de la capsule. A la figure 4, le mouvement s'est terminé et la capsule est fermée, le compartiment intérieur étant rempli de matière intestinale (l'échantillon).

L'enrobage 2 est choisi pour une dissolution dans l'intestin en un temps prédéterminé, afin que la prise d'échantillon se fasse dans le compartiment intestinal voulu. On prévoit notamment de réaliser plusieurs lots de capsules avec des enrobages spécifiques pour chaque lot, conduisant à des vitesses de dissolution différentes. Il est ensuite possible d'administrer à l'individu des capsules de ces différents lots, afin de prélever des échantillons à différents endroits prédéterminés dans l'intestin.

Avantageusement, les capsules de chaque lot ont une particularité (par exemple couleur des disques) permettant de les identifier.

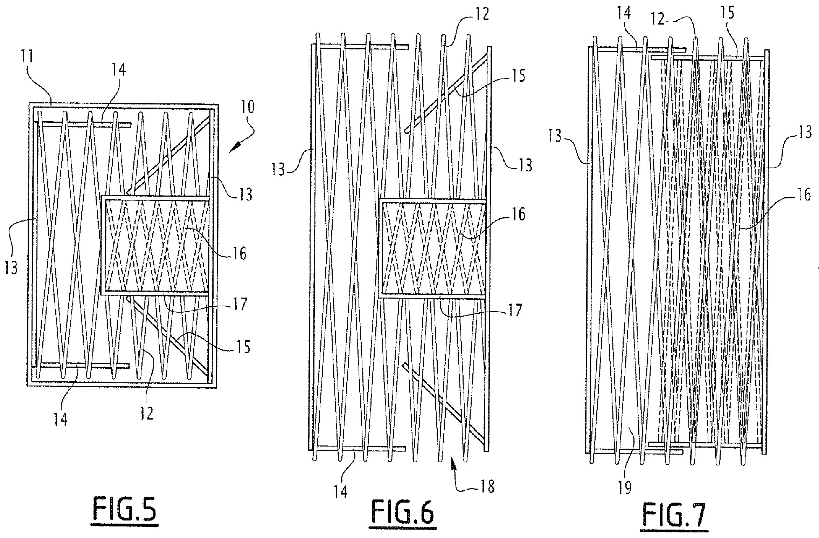

La figure 5 représente une vue schématique d'une capsule maillée 10 cylindrique contrainte dans un enrobage 11 dissolvable dans l'intestin, selon un deuxième mode de réalisation. Les dimensions peuvent être les mêmes que celles décrites pour le mode de réalisation de la figure 1, pour un ensemble de petites capsules pouvant être réunies dans une plus grande capsule pour l'administration orale, ou pour une capsule de plus grande dimension destinée à être administrée directement. De même, ces capsules et leur enrobage dissolvable dans l'intestin peuvent être comme décrit pour le mode de réalisation précédent, de manière à pouvoir disposer de capsules ayant des enrobages à vitesses de dissolution différentes.

La capsule comprend, contrainte dans le matériau dissolvable dans l'intestin, une structure maillée tubulaire extensible dans les deux directions orthogonales (dans l'axe du cylindre et de manière rayonnée). Cette structure maillée est formée d'un stent 12 contraint dans les deux directions (figure 5) et déployé (figures 6 et 7), entre deux disques 13 formant les bases de la forme cylindrique de la capsule. L'un des disques 13 est solidaire d'une paroi cylindrique 14 formant une partie cylindrique de délimitation du compartiment de réception d'échantillon. L'autre disque 13 est solidaire d'une deuxième paroi cylindrique 15 ayant, au repos, une forme de cône tronqué dirigé vers le centre du compartiment. Les disques et parois sont en matériau souple extensible, par exemple un élastomère ou un tissu compressé (e.g. froncé), ayant par exemple des pores perméables à l'eau et arrêtant les microorganismes, notamment les bactéries. Les deux parois 14 et 15 sont situées à l'intérieur de la structure maillée. Un deuxième stent 16, plus petit que le précédent, est placé dans le compartiment au droit de la paroi 14, provisoirement fixé à cette paroi par une colle se dégradant rapidement au contact du liquide intestinal (par exemple PVA, Polyméthacrylates, amylose, HPMC...). Ce stent est conçu de manière à se déployer de manière rayonnée seulement. Ce stent est contraint par un matériau dégradable dans le milieu intestinal.

Lors de l'administration à un individu, la capsule est repliée comme représentée à la figure 5. Dans l'intestin, la couche de matériau 11 va se dissoudre entraînant le déploiement dans les deux directions du stent 12 et, sous son action, des disques 13 et de la paroi 14. Le milieu ou liquide intestinal peut alors entrer dans le compartiment ainsi esquissé par l'ouverture 18. Sous l'effet de ce liquide entrant dans le compartiment, la couche de matériau 17 est à son tour dissoute, ce qui entraîne le déploiement rayonné du stent 16 qui va venir s'appliquer à la face intérieure de la paroi 15, la repoussant vers l'extérieur, jusqu'à ce qu'elle vienne s'appuyer contre la paroi 14 sur une zone de superposition et ferme le compartiment 19 (figure 7). On peut prévoir en plus une fermeture par scratch et/ou aimantation pour optimiser la fermeture du compartiment 19.

A titre d'exemple, la longueur de la capsule est de 2 mm et sa hauteur (diamètre du cylindre) de 4,5 mm à la figure 5, puis passe à 4 mm de longueur et 9 mm de hauteur à la figure 6 et le volume du compartiment est alors de 254 µl.

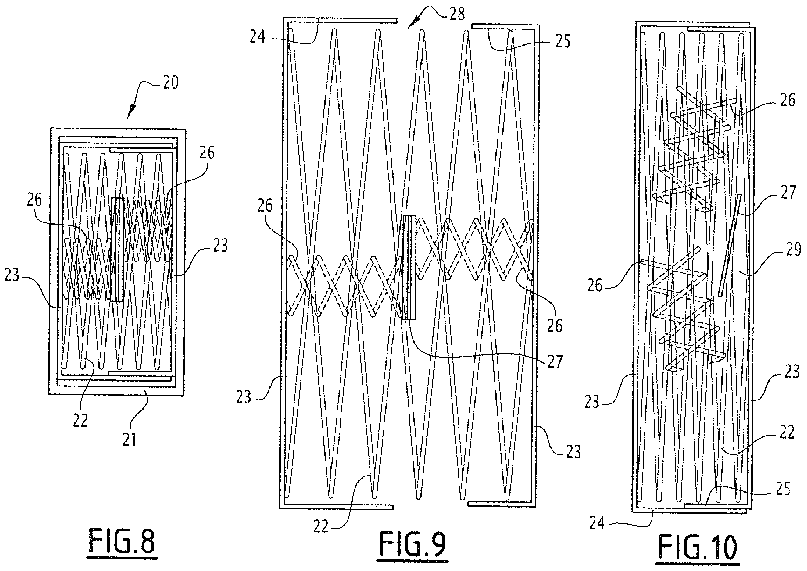

La figure 8 représente une vue schématique d'une capsule maillée 20 cylindrique contrainte dans un enrobage 21 dissolvable dans l'intestin, selon un troisième mode de réalisation. Les dimensions peuvent être les mêmes que celles décrites pour le mode de réalisation de la figure 1, pour un ensemble de petites capsules pouvant être réunies dans une plus grande capsule pour l'administration orale, ou pour une capsule de plus grande dimension destinée à être administrée directement. De même, ces capsules et leur enrobage dissolvable dans l'intestin peuvent être comme décrit pour le mode de réalisation précédent, de manière à pouvoir disposer de capsules ayant des enrobages à vitesses de dissolution différentes.

La capsule comprend, contrainte dans le matériau dissolvable dans l'intestin, une structure maillée tubulaire extensible dans les deux directions orthogonales (dans l'axe du cylindre et de manière rayonnée). Cette structure maillée est formée d'un stent 22 contraint dans les deux directions (figure 8) et déployé (figures 9 et 10), entre deux disques 23 formant les bases de la forme cylindrique de la capsule. Les deux disques 23 sont solidaires chacun d'une paroi cylindrique 24, respectivement 25, formant deux parties cylindriques de délimitation du compartiment de réception d'échantillon. Les disques et parois sont en matériau souple extensible, par exemple un élastomère ou un tissu compressé (e.g. froncé), ayant par exemple des pores perméables à l'eau et arrêtant les microorganismes, notamment les bactéries. Les parois cylindriques portées par les disques sont situées à l'extérieur par rapport à la structure maillée. Deux ressorts 26 sont placés dans le compartiment au droit des disques 23 auxquels ils sont fixés de manière provisoire par un polymère rapidement dissout par le liquide intestinal (voir exemple précédent), les deux ressorts étant fixés ensemble au centre du compartiment, par l'intermédiaire d'un disque 27 de part et d'autre duquel les ressorts s'appuient en opposition de manière décalée, et d'une colle rapidement dissoute par le liquide intestinal. Ces ressorts 26 ont une force supérieure à celle du stent 22 dans la direction axiale, on verra que cela leur permet de forcer le déploiement du stent dans la direction axiale au-delà de la position de repos du stent 22 déployé.

Lors de l'administration à un individu, la capsule est repliée comme représentée à la figure 8. Dans l'intestin, la couche de matériau 21 va se dissoudre entraînant le déploiement dans les deux directions du stent 22, et en même temps des disques 23 et des parois 24 et 25. Cela permet aussi le déploiement des ressorts 26 en direction axiale, contraignant le stent 22 au-delà de sa position de repos. Les dimensions des parois 24 et 25 sont telles que, tant que les ressorts sont déployés, leurs bords en regard ne sont pas au contact offrant une ouverture 28 pour l'entrée de liquide intestinal dans le compartiment (figure 9). Sous l'effet de ce liquide entrant dans le compartiment, la colle immobilisant les ressorts 26 se dissout, libérant ces derniers, puis le décalage d'appui des ressorts sur le disque 27 conduit les ressorts à se désengager l'un de l'autre rendant sa liberté au stent 22 qui prend sa position déployée de repos, conduisant les parois 24 et 25 à venir en contact pour fermer le compartiment 29 qui renferme alors l'échantillon (figure 10).

A titre d'exemple, la longueur de la capsule est de 2 mm et sa hauteur (diamètre du cylindre) de 4,5 mm à la figure 8, puis passe à 4 mm de longueur et 9 mm de hauteur à la figure 6 et enfin à 2 mm de longueur et 9 mm de hauteur à la figure 10. Volume du compartiment : 127 µl.

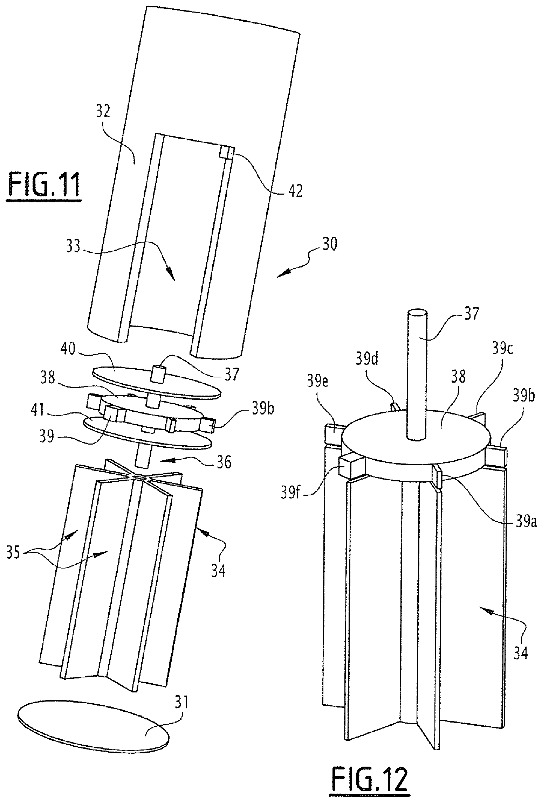

La figure 11 représente une vue schématique éclatée d'une capsule cylindrique selon un quatrième mode de réalisation. Cette capsule 30 comporte, de haut en bas, une base 31, une enveloppe 32 avec fente verticale 33, un carrousel 34 muni de plusieurs compartiments rayonnés 35 et d'un orifice central vertical 36, un arbre d'entraînement 37 raccordé à une pièce 38. Une fois l'ensemble monté, la fente 33 peut se présenter successivement devant les compartiments 35 pour que du liquide intestinal puisse être prélevé, et l'arbre d'entraînement 37 vient s'accoupler à l'intérieur de l'orifice central du carrousel 34. La figure 12 montre un carrousel ou barillet de 6 compartiments..

Diverses solutions d'entraînement en rotation du carrousel sont envisageables. Un premier est un dispositif mécanique d'entraînement en rotation de l'arbre 37, par exemple de type horloger ou compte-minutes de cuisine, miniaturisé.

Un deuxième est que l'arbre 37 soit un fil à torsion, ou à mémoire de forme, qui a tendance à vouloir retrouver sa position de repos ou sa forme initiale. Dans ce cas, il convient de freiner cette propension de manière que la rotation du carrousel et la présentation d'un compartiment 35 devant la fente 33 soit contrôlée et conforme au processus de prise d'échantillon au sein de l'intestin.

Dans le mode de réalisation des figures 11 et 12, le dispositif d'entraînement en rotation est formé de l'association d'un fil ressort de torsion 37, d'un disque 38 portant, en périphérie, des freins 39a-39f de dimensions croissantes (donc impliquant des temps de dissolution différents), le disque étant entouré de flasques d'étanchéité 40 et 41. L'enveloppe 32 porte au coin supérieur de sa fente 33, une butée fixe 42 sur laquelle un frein vient buter, puis, lorsque ce frein est dégradé, le fil ressort de torsion 37 fait tourner le barillet d'un pas (d'un compartiment) jusqu'à ce que le frein suivant vient s'appuyer sur la butée 42.

A titre d'exemple, une première capsule assemblée fait 25 mm de hauteur pour un diamètre de 12 mm, une deuxième fait 12 mm de hauteur et 10 mm de diamètre. On peut faire varier ces dimensions dans des limites rendant l'ingestion orale possible. On peut ensuite faire varier le nombre de compartiments (par exemple de 5 à 20) et, à taille égale, le volume de chaque compartiment se réduit à mesure que le nombre augmente.

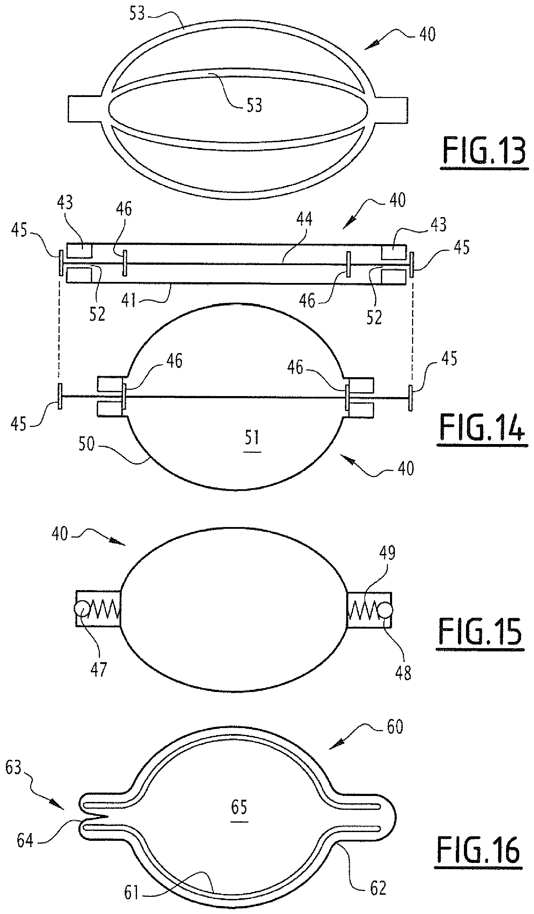

Les figures 13-15 représentent une vue schématique d'une capsule selon un cinquième mode de réalisation, fonctionnant avec un principe d'aspiration du liquide intestinal, mais sans qu'il soit indispensable de mettre le compartiment interne sous basse pression à la fabrication de la capsule.

La figure 14, vue supérieure, montre la capsule 40 en son état initial, tel qu'administré à un individu. Il s'agit d'un tube 41 en alliage NiTi découpé longitudinalement (par exemple au laser) sauf aux extrémités où subsistent deux parties d'extrémité 42 et soumis à un traitement lui conférant une mémoire de forme déployée comme à la figure 13 (structures avec barreaux 53). Des paliers cylindriques 43 sont fixés à l'intérieur des parties d'extrémité et une tige 44 s'étend axialement dans le tube 41 et les paliers 43. Cette tige dépasse à l'extérieur du tube 41 et se termine à ses deux extrémités par un tampon d'étanchéité 45, par exemple en élastomère. Deux autres tampons d'étanchéité 46 similaires sont placés sur la tige, à l'intérieur du tube et à proximité des paliers 43. Le tube 41 est entouré d'une membrane (schématiquement représentée à la figure 14, repère numérique 50) en matériau silicone souple et résistant au milieu intestinal. Sont ensuite appliqués autour de cette membrane, une ou plusieurs bandes (non représentées) en matériau dégradable dans l'intestin, qui contraint la capsule dans la forme tubulaire (figure 14, en haut). Après dissolution de cette ou ces bandes, le tube 41 tend à prendre sa forme déployée, les tampons 45 n'assurent plus alors l'étanchéité au niveau des paliers, le liquide intestinal étant aspiré par la lumière 52 des paliers formant ouverture d'admission. Lorsque la capsule est entièrement déployée, les tampons 46 viennent au contact des paliers et obturent la capsule, contenant l'échantillon.

La figure 15 présente une variante plus simple, où les parties d'extrémité de la capsule logent des valves 47 de type valve de ballon avec ressort 48 et bille 49 d'étanchéité, permettant seulement l'admission de liquide intestinal dans le compartiment 51, lors du déploiement de celle-ci après dégradation de la ou des bandes en matériau dégradable.

A titre d"exemple, les dimensions sont choisies pour un volume de compartiment de 150 à 300 µl. Par exemple, on peut partir d'un cylindre 41 de 6 mm de diamètre et de 8 mm de longueur, pour obtenir un volume d'échantillon aspiré d'environ 150 µl dans le tube déployé.

Dans un autre mode de réalisation, à la figure 16, le corps de la capsule 60 est formé d'une cage 61 par exemple en matériau à mémoire de forme, e .g. en alliage NiTi ayant naturellement (au repos) une forme ovoïde. La cage est enveloppée d'une membrane 62 souple et élastique, par exemple en latex ou en silicone ajustée à la forme de la cage au repos. La membrane obstrue totalement une des deux ouvertures et laisse l'autre 63 ouverte, comme le ferait un doigt de gant recouvrant la cage. Au niveau de l'extrémité ouverte, la membrane souple est retournée sur elle-même comme un doigt de gant, et est introduite à l'intérieur de la cage. Dans ce mode de réalisation, la partie 64 de la membrane située à l'intérieur joue le rôle d'une valve anti-retour 64. Sont ensuite appliqués autour de cette membrane, une ou plusieurs bandes (non représentées) en matériau dégradable dans l'intestin, qui contraint la capsule dans une forme tubulaire. Après dissolution de cette ou ces bandes, la cage 60 tend à prendre sa forme déployée Le liquide intestinal est aspiré à l'intérieur 65 de la capsule au moment du gonflement de la cage, il passe librement l'ouverture 63 en écartant la membrane. Une fois la capsule remplie, le liquide ne peux ressortir puisque l'élasticité et la forme de la membrane constitue cette fois une barrière au liquide. La pression interne du liquide tend à gonfler la membrane en 64, qui forme des structures arrondies se faisant face et obstruant l'ouverture.

Des vitesses de dégradation différentes peuvent être conférées aux matériaux dissolvables utilisables dans les exemples qui précèdent et les autres formes de réalisation possibles de l'invention. Ces vitesses de dégradation contrôlées peuvent être obtenues comme cela est connu de l'homme du métier, par exemple par le choix du matériau, par exemple nature du matériau, du polymère, présence ou pas d'additifs jouant sur la solubilité du matériau, du polymère ou d'un composant, par exemple un liant, composition pondérale lorsqu'il y a plusieurs composants, nature des enzymes de dégradation dans le cas où la dégradation se fait de manière enzymatique, et/ou l'épaisseur du matériau.

Comme on l'a vu, il peut y avoir déjà une couche ou enveloppe extérieure de matériau gastro-résistant. Il peut s'agir notamment d'un revêtement ou d'une capsule dans laquelle on insère le dispositif.

Il peut ensuite y avoir une ou des couches ou un ou des freins ou autres dispositifs dissolvables, dont la dissolution va permettre d'ouvrir un compartiment de prise d'échantillon et/ou de déclencher une prise d'échantillon.

Pour les modes de réalisation utilisant des couches ou enveloppes de maté ériaus, on peut apporter les précisions suivantes.

A/Enrobage en une seule couche :
La qualité, la quantité, le degré de substitution du/des polymère (s), et l'épaisseur de la couche peuvent permettre de moduler la durée nécessaire pour activer ou libérer la fonction. La couche peut être composée :
- D'un polymère gastro-résistant et soluble au pH de l'intestin grêle et dans lequel sont intégrés d'autres polymères, excipients qui permettent de moduler la durée d'érosion de l'enrobage (comme indiqué dans le point B ci-dessous).
- D'un polymère insoluble dans le tractus gastro-intestinal (GIT) et non érodable (ex : ethylcellulose, acrylate et methacrylate...) dont on contrôle la porosité et ainsi la diffusion de l'eau, la dilatation du dispositif, e.g. stent déclenchera un éclatement de la couche d'enrobage.
- D'un mélange de polymères Insolubles et solubles dans le GIT (ex : un mélange Ethyl cellulose- Hydroxypropylmethylcellulose HPMC).

B/Enrobage en plusieurs couches :
1- Une couche extérieure :
   On peut utiliser :
   - Un film ou une enveloppe gastro-résistant dont la solubilité dépend du pH type, par exemple, acetophtalate de cellulose, certains copolymère d'ester de méthacrylate, etc., et qui ne se dissolvent qu'à des pH basiques.
   - Une membrane insoluble dans GIT semi-perméable mais qui peut se rompre sous la pression induite par des agents effervescents, superdésintégrant (Croscaramellose), d'osmose (NaCI) ou des polymères qui gonflent au contact de l'eau (HPMC).
2- Couches intérieures :
   Il peut y avoir une ou plusieurs couches intérieures composées d'un ou plusieurs polymères dont la désintégration peut être activée et contrôlée de plusieurs façons. La qualité du polymère, nombre et l'épaisseur des couches peut permettre de moduler la durée nécessaire pour activer le dispositif.

La désintégration de l'enrobage peut se faire en utilisant :
- Des polymères qui s'érodent par dissolution, par digestion enzymatique, ou par tout autre stimuli tel que la variation du pH
- Des polymères hydrophiles tels que dérivées de la cellulose hyroxypropyl methylcellulose (HPMC), hydroxyethylcellulose (HEC) and hydroxypropyl cellulose (HPC) qui gonflent et s'érodent au contact de l'eau. Le contrôle de la vitesse de gonflement permet de contrôler le temps nécessaire pour que l'eau atteigne le dispositif et active sa dilatation.

## Revendications

1. Capsule (40, 60) destinée au prélèvement d'échantillon dans l'intestin, comprenant un corps (41, 50) délimitant au moins un compartiment (51, 65) de réception d'échantillon, un matériau dissolvable dans l'intestin entourant totalement ou partiellement le corps, ledit corps comprenant un dispositif (53 ; 61) initialement contraint sous une forme repliée par ledit matériau dissolvable dans l'intestin et dont le déploiement entraîne le déploiement du corps, au moins une ouverture (52, 63) d'admission d'échantillon, et un mécanisme (44 ; 64) mobile d'ouverture et/ou de fermeture de l'ouverture, le déploiement du dispositif et du corps et l'actionnement du mécanisme étant commandés par la dissolution du matériau dissolvable dans l'intestin,
**caractérisée en ce que** le dispositif (53 ; 61) est une cage à mémoire de forme, ladite cage comprenant une structure de base entourée d'une membrane (50, 62) étanche, souple et élastique, la cage ayant naturellement une forme ovoïde.

2. Capsule selon la revendication 1, dans laquelle ledit mécanisme est un mécanisme (44) mobile d'ouverture et de fermeture de l'ouverture.

3. Capsule selon l'une des revendications 1 et 2, dans laquelle la cage est initialement contrainte par le matériau dissolvable dans l'intestin, ladite structure présentant en outre une ouverture (52) à ses extrémités et pour chaque ouverture un tampon (46) d'étanchéité associé audit mécanisme mobile pour assurer la fermeture de ces ouverture (52) seulement lorsque la structure s'est déployée.

4. Capsule selon la revendication 3, dans laquelle le mécanisme comprend une tige (44) s'étendant axialement au travers de la structure, des paliers (43) placés dans ces ouvertures, chaque palier ayant un orifice central (52) de diamètre supérieur au diamètre de la tige, les tampons (46) d'étanchéité étant montés sur la tige à un emplacement les amenant en appui sur les paliers lorsque la structure a pris sa forme ovoïde.

5. Capsule selon l'une des revendications 1 et 2, dans laquelle la cage est initialement contrainte par un matériau dissolvable dans l'intestin, ladite structure présentant en outre au moins une ouverture (63) à une extrémité et une valve anti-retour placée dans l'ouverture (63).

## Patentansprüche

1. Kapsel (40, 60), die zur Probenentnahme im Darm vorgesehen ist, umfassend einen Körper (41, 50), der mindestens eine Kammer (51, 65) zum Erhalt einer Probe abgrenzt, ein im Darm lösliche Material, das den Körper vollständig oder zum Teil umgibt, wobei der Körper eine Vorrichtung (53; 61) umfasst, die zunächst durch das im Darm lösliche Material in einer gefalteten Form begrenzt ist und deren Entfaltung die Entfaltung des Körpers bewirkt, mindestens eine Öffnung (52, 63) zur Aufnahme einer Probe und einen beweglichen Mechanismus (44; 64) zum Öffnen und/oder zum Schließen der Öffnung, wobei die Entfaltung der Vorrichtung und des Körpers und die Betätigung des Mechanismus durch das Lösen des im Darm lösbaren Materials gesteuert werden,
**dadurch gekennzeichnet, dass** die Vorrichtung (53; 61) ein Formgedächtniskäfig ist, wobei der Käfig eine Basisstruktur umfasst, die von einer dichten, weichen und elastischen Membran (50, 62) umschlossen wird, wobei der Käfig naturgemäß eine ovale Form aufweist.

2. Kapsel nach Anspruch 1, wobei der Mechanismus ein beweglicher Mechanismus (44) zum Öffnen und zum Schließen der Öffnung ist.

3. Kapsel nach einem der Ansprüche 1 und 2, wobei der Käfig zunächst durch das im Darm lösbare Material begrenzt ist, wobei die Struktur weiterhin eine Öffnung (52) an ihren Enden und für jede Öffnung einen Dichtstopfen (46) aufweist, der mit dem beweglichen Mechanismus assoziiert ist, um das Schließen dieser Öffnung (52) nur, wenn die Struktur entfaltet wird, sicherzustellen.

4. Kapsel nach Anspruch 3, wobei der Mechanismus eine Stange (44), die sich axial durch die Struktur erstreckt, Lager (43), die in diesen Öffnungen angeordnet sind, umfasst, wobei jedes Lager ein zentrales Loch (52) mit einem Durchmesser aufweist, der größer als der Durchmesser der Stange ist, wobei die Dichtstopfen (46) auf der Stange an einer Stelle montiert sind, der sie in Auflage auf den Lagern bringt, wenn die Struktur seine ovale Form angenommen hat.

5. Kapsel nach einem der Ansprüche 1 und 2, wobei der Käfig zunächst durch ein im Darm lösbares Material begrenzt ist, wobei die Struktur weiterhin mindestens eine Öffnung (63) an einem Ende und ein Rückschlagventil, das in der Öffnung (63) angeordnet ist, aufweist.

## Claims

1. Capsule (40, 60) intended to collect samples in the intestine, comprising a body (41, 50) delimiting at least one compartment (51, 65) for receiving samples, a material that dissolves in the intestine totally or partially surrounding the body, said body comprising a device (53; 61) initially constrained in folded up form by said material that dissolves in the intestine and the deployment of which triggers the deployment of the body, at least one sample intake opening (52, 63), and a mobile mechanism (44; 64) for opening and/or closing the opening, the deployment of the device and the body and the actuation of the mechanism being controlled by the dissolution of the material that dissolves in the intestine,
**characterised in that** the device (53; 61) is a shape-memory cage, said cage comprising a base structure surrounded by an impervious, flexible and elastic membrane (50, 62), the cage having naturally an ovoid shape.

2. Capsule according to claim 1, wherein said mechanism is a mobile mechanism (44) for opening and closing the opening.

3. Capsule according to one of claims 1 and 2, wherein the cage is initially constrained by the material that dissolves in the intestine, said structure further having an opening (52) at the ends thereof and for each opening a sealing buffer (46) associated with said mobile mechanism to ensure the closure of these openings (52) only when the structure is deployed.

4. Capsule according to claim 3, wherein the mechanism comprises a rod (44) extending axially through the structure, bearings (43) placed in these openings, each bearing having a central orifice (52) of greater diameter than the diameter of the rod, the sealing buffers (46) being mounted on the rod at a location bringing them to bear against the bearings when the structure has taken the ovoid shape thereof.

5. Capsule according to one of claims 1 and 2, wherein the cage is initially constrained by a material that dissolves in the intestine, said structure further having at least one opening (63) at one end and a one-way valve placed in the opening (63).
